Europäisches Patentamt

European Patent Office

Office européen des brevets

(11) Publication number: **0 339 923 B1**

# EUROPEAN PATENT SPECIFICATION

(45) Date of publication of patent specification: **12.01.94** (51) Int. Cl.⁵: **C07C 1/04**, B01J 23/74

(21) Application number: **89304092.3**

(22) Date of filing: **25.04.89**

(54) **Process for hydrocarbon synthesis catalyzed by cobalt on titania, enhanced by water addition.**

(30) Priority: **25.04.88 US 185953**

(43) Date of publication of application:
**02.11.89 Bulletin 89/44**

(45) Publication of the grant of the patent:
**12.01.94 Bulletin 94/02**

(84) Designated Contracting States:
**DE GB NL SE**

(56) References cited:
**EP-A- 0 109 702**
**EP-A- 0 216 967**
**EP-A- 0 266 898**
**FR-A- 2 388 782**
**US-A- 4 595 703**

(73) Proprietor: **EXXON RESEARCH AND ENGI-
NEERING COMPANY**
**P.O.Box 390,**
**180 Park Avenue**
**Florham Park, New Jersey 07932-0390(US)**

(72) Inventor: **Kim, Chang Jung**
**RR No. 1, Rattlesnake Bridge Road**
**Bedminster New Jersey 07921(US)**

(74) Representative: **Somers, Harold Arnold et al**
**ESSO Engineering (Europe) Ltd.**
**Patents & Licences**
**Mailpoint 72**
**Esso House**
**Ermyn Way**
**Leatherhead, Surrey KT22 8XE (GB)**

Rank Xerox (UK) Business Services
(3.10/3.9/3.3.3)

**Description**

The present invention relates to the Fischer-Tropsch catalysed synthesis of hydrocarbons from a gaseous feed mixture of $H_2$ and CO. The problem to which the invention is directed is to increase the total CO conversion yield without sacrifice of selectivity in the yield of heavier hydrocarbons, especially $C_5$ + but also $C_{10}$ + hydrocarbons.

EP-A-109,702 describes a process in which 10-40 vol.% of steam is added to the feed mixture of $H_2$ and CO, at elevated temperature and pressure, using a catalyst comprising 10-40 pbw cobalt and 0.1-150 pbw of zirconium, titanium or chromium per 100 pbw silica support, the catalyst having been prepared by impregnation and/or kneading. The publication describes some improvement in terms of $C_3$ + selectivity using a zirconium promoted cobalt catalyst, but with no significant increase, and more usually a decrease in total CO conversion yield. The results indicate no improvement using cobalt alone.

According to the present invention there is provided a process for synthesising $C_5$ + hydrocarbons comprising feeding $H_2$ and CO together with $H_2O$ into contact with a catalyst composition in a reaction zone a temperature of at least 100°C and a pressure above atmospheric, wherein said catalyst composition comprises cobalt, optionally promoted with one or more promoter metals selected from Re, Hf, Nb, Ta, Cr, Zn, and lanthanide elements, supported on titania.

By cofeeding $H_2O$ into the reaction zone, along with the $H_2$ and CO, it has been found that $C_5$ + hydrocarbon production is increased, CO conversion is increased and $CH_4$ production is decreased. The cobalt/titania catalyst may be promoted with one or more suitable promoter metals, such as rhenium.

The $H_2O$ that is added to the reaction zone may be in the form of steam or moisture or a suitable $H_2O$ precursor, such as $C_1$-$C_6$ alcohols, for forming $H_2O$ in-situ in the reaction zone. It is essential to the understanding of the process of this invention that the $H_2O$ introduced into the reaction zone is external $H_2O$ and not that $H_2O$ which is formed in-situ in the reaction zone as a consequence of the Fischer-Tropsch hydrocarbon synthesis reaction from the $H_2$ and CO. It has also been found that the process of this invention improves with increasing pressure in the reaction zone and, in terms of $C_5$ + productivity, with decreasing CO conversion.

DETAILED DESCRIPTION

The process of the present invention resides in adding, to the Fischer-Tropsch reaction zone, $H_2O$ or a suitable $H_2O$ precursor such as an alcohol. The amount of $H_2O$ added to the reaction zone will generally range from 1-70 volume % of the total feed mixture of $H_2O$, CO and $H_2$ and, preferably, from 5-30 volume %. As the extent of CO conversion increases, the amount of water produced in-situ in the reaction zone increases and, concomitantly the beneficial effect of introducing additional water into the reaction zone to increase CO conversion, reduce $CH_4$ selectivity and increase $C_5$ + hydrocarbons selectivity decreases. Thus, in some cases, it may be advantageous to practice the process of this invention CO conversion levels below 60 percent. That is, below 60% per pass, reaction zone or stage. As the pressure in the reaction zone increases, the beneficial effect of the process of this invention of adding water to the reaction zone increases with respect to increasing CO conversion activity, decreased $CH_4$ selectivity and increased $C_5$ + hydrocarbon selectivity. At relatively low pressures in the reaction zone (i.e., less than atmospheric), little effect will be seen in increased conversion activity, etc. by adding $H_2O$ to the reaction zone. Thus, the process of this invention will be operated at a pressure above atmospheric. In general the pressure will range from above atmospheric to 5.06 MPa (50 atmospheres), and more preferably 506 kPa - 3.04 MPa (5-30 atmospheres).

In the process of this invention, the addition of $H_2O$ enhances the formation of $C_5$ + hydrocarbons and reduces $CH_4$ make in a uni-directional or linear fashion. It has also been found that the water addition of the process of this invention enhances olefin formation. Further, it has been found that the rate of the Fischer-Tropsch reaction employing the process of this invention increases with increasing partial pressure of $H_2O$ at any fixed total pressure in the reaction zone, up to a point, after which point the rate slowly goes down with continually increasing $H_2O$ partial pressure. As an illustrative, but non-limiting example, in a plug flow reactor operated at 200°C and 2.03 MPa (20 atmospheres) of synthesis gas pressure, with a $H_2$/CO ratio of 2 and at low CO conversion conditions using a rhenium promoted cobalt/$TiO_2$ catalyst, the inlet $H_2O$ partial pressure was systematically varied from 0 (no external $H_2O$ addition) to 2.13 MPa (21 atmospheres). The productivity of the catalyst increased with increasing $H_2O$ pressure up to the point when the $H_2O$ pressure reached about 709 kPa (7 atmospheres), where a 3 fold higher activity was observed as compared with the base case without $H_2O$ addition. Further increase in $H_2O$ pressure induced a gradual decrease in activity and eventually, at an $H_2O$ partial pressure of 2.13 MPa (21 atmospheres), the productivity was only 50%

2

higher than the base case with no external water addition.

In general, the Fischer-Tropsch hydrocarbon synthesis reaction process of this invention is carried out at a $H_2$:CO mole ratio of greater than 0.5, and preferably the $H_2$:CO mole ratio ranges from 0.5 to 6, more preferably from 0.5 to 3, at gas hourly space velocities ranging from 100 V/Hr/V to 5000 V/Hr/V, preferably from 300 V/Hr/V to 1500 V/Hr/V, at temperatures ranging from 150°C to 300°C, preferably from 180°C to 240°C, and pressures above atmospheric, preferably ranging up to 5.06 MPa (50 atm.), more preferably from 5 atm. to 40 atm. and still more preferably from 506 kPa - 3.04 MPa (5-30 atmospheres). In its most preferred form, a bed of catalyst comprised of from 5 percent to 15 percent cobalt, containing a suitable promoter such as rhenium in an amount sufficient to provide a catalyst containing rhenium:cobalt in ration ranging from 0.025:1 to 0.10:1, is dispersed on titania, preferably a high purity titania, and a bed of such catalyst is contacted with a gaseous admixture of carbon monoxide and hydrogen, or compound decomposable in-situ within the bed to generate carbon monoxide and hydrogen.

As previously stated, the hydrocarbon synthesis process of this invention employs a catalyst comprising cobalt supported on $TiO_2$. The catalyst will be a particulate catalyst composition containing a catalytically active amount of cobalt. The cobalt may, if desired, be promoted with one or more suitable promoter metals. In one embodiment, the promoter metal will be selected from the group consisting of Re, Hf, V, Nb, Ta, Cu, Zn, the lanthanide series elements or mixture thereof. Preferably the promoter will comprise Re alone or with one or more of the aforementioned elements. The promoter, if used, is preferably added to the catalyst in an amount to form a catalyst having a promoter:cobalt weight ratio greater than 0.010:1, preferably from 0.025:1 to 0.10:1. In terms of absolute concentrations, from 0.05 percent to 3 percent, preferably from 0.15 percent to 1 percent of promoter, based on the total weight of the catalyst composition (dry basis), is dispersed with the catalytically active amount of cobalt on a titania support. In terms of absolute of concentrations, cobalt is preferably present in the composition in amounts ranging from 2 percent to 25 percent, preferably from 5 percent to 15 percent, based on the total weight of the catalyst composition (dry basis), and sufficient promoter, such as rhenium, is optionally added to form a catalyst having a weight ratio of rhenium:cobalt greater than 0.010:1, preferably from 0.025:1 to 0.10:1, based on the total weight of the cobalt and rhenium contained in the catalyst composition (dry basis). The absolute concentration of each metal will, of course, be preselected to provide the desired ratio of rhenium:cobalt as heretofore expressed.

The promoted or unpromoted cobalt/titania catalysts useful in the practice of this invention may be prepared by various techniques well known to those skilled in the art. In general, the cobalt or cobalt and promoter metal, will be deposited or impregnated onto a previously pilled, pelleted, beaded, extruded or sieved titania support material by the impregnation method which also includes the incipient wetness technique. The procedure set forth below, although specifically directed to rhenium as the promoter metal, also applies to other promoter metals and mixtures of promoter metals.

In preparing a rhenium promoted catalyst, the cobalt and rhenium metals are deposited from solution onto the support material in preselected amounts in order to provide the desired amounts of cobalt and rhenium metals and weight ratio of the respective metals of cobalt and rhenium and the metal loading of the catalytic metals onto the titania support. In one method, the cobalt and rhenium are composited with the titania support material by contacting the support with a solution of a cobalt-containing compound, or salt, or a rhenium-containing compound, or salt, e.g., a nitrate, carbonate or the like. The cobalt salt or precursor may first be impregnated on the support followed by impregnation of the rhenium salt or compound or they may be impregnated simultaneously. The cobalt and rhenium precursor compounds used in the impregnation can be any organometallic or inorganic compounds which decompose to give cobalt and rhenium oxides upon calcination, such as a cobalt or rhenium nitrate, acetate, nitrite, carbonate, acetylactonate, naphthenate, carbonyl, or the like. The amount of impregnation solution used should be sufficient to completely wet or immerse the support material, usually within the range from 1 to 20 times of the carrier by volume, depending on the metal, or metals, concentration in the impregnation solution.

The impregnation treatment can be carried out under a wide range of conditions including ambient or elevated temperature. Metal components other than cobalt and rhenium can also be added. The introduction of additional promoter metal, or metals, into the catalyst can be carried out by any method and at any time of the catalyst preparation, for example, prior to, following or simultaneously with the impregnation of the titania support with the cobalt and rhenium components.

The catalyst precursor formed by impregnation of the titania support material by the catalytic metal components, will generally be dried by heating at a temperature above 30°C, preferably above 125°C, in the presence of a suitable atmosphere such as air, nitrogen, oxygen, etc. or under vacuum. It is normally necessary to calcine the catalyst precursor composite prior to use in order to convert the rhenium and cobalt to their respective oxides. Thus, the catalyst precursor composite will normally be contacted with oxygen, air, or other oxygen-containing gas at a temperature sufficient to oxidize the rhenium and cobalt and convert same to their respective oxides. This may be done at temperatures above 150 and preferably above 200°C. However, temperatures up to 500°C may also be used to form, and if necessary, regenerate a severely deactivated catalyst.

In general, the catalyst precursor will be calcined at temperatures ranging from 150 to 300°C. After the catalytic metals have been oxidized, the cobalt and rhenium oxides contained on the catalyst are normally reduced to form the final catalyst composition useful in the process of this invention. This reduction is normally accomplished by contacting the catalyst, whether or not previously oxidized, with a suitable reducing gas such as hydrogen or a hydrogen-containing gas stream at elevated temperatures above 250°C and preferably above 300°C. Generally the catalyst is fascile ranging from 250 to 500°C and preferably from 300 to 450°C for periods ranging from 1/2 to 24 hours at pressures ranging from ambient to 4.05 MPa (40 atmospheres).

The invention will be more readily understood by reference to the examples below.

## EXAMPLES

### Example 1 - 12% $Co/TiO_2$

High purity $(Co(NO_3)_2 \cdot 6H_2O$ (99.999% purity puratronic grade, 25.0 grams) was dissolved in 18 ml of spectroscopic grade acetone and this solution was added dropwise, while stirring, to 37 grams of rutile $TiO_2$ (80-140 mesh, BET = 11 $m^2$/g). The resulting material was dried overnight at 90°C, followed by calcination in air at 250°C for 16 hours. Reduction was carried out in $H_2$ (500 $scm^3$/m, atmospheric pressure) by increasing the temperature to 450°C over a period of 4 hours and then keeping at 450°C for 16 hours. The reduced catalyst was then passivated at 25°C using a 1% $O_2$ - 99% He mixture. Chemisorption measurements showed that this $Co/TiO_2$ catalyst had 2.9% dispersion (2.9% of the Co was exposed).

The performance test of this catalyst was carried out by charging an intimate mixture of the said 12% $Co/TiO_2$ catalyst (3.0 grams) and a diluent (low-surface-area rutile, 80-140 mesh, 18 grams) into a down-flow fixed bed reactor made of 96mm (3/8") OD stainless steel tube with a concentric 32mm (1/8") OD thermocouple well. The use of diluent and an aluminum block jacket fitted tightly around the reactor minimized uneven temperature profile along the bed. The catalyst was then rereduced in a flowing $H_2$ stream (200 cc/m, atmospheric) overnight at 450°C, cooled to 175°C and then pressurized to 2.03 MPa (20 atm) using a pre-mixed gas composed of 63.9% $H_2$, 32.1% CO and 4.0% $N_2$. At pressure and at a preset flow rate, the temperature was raised to 200°C over a period of one hour and then data acquisition was initiated. The rate of CO conversion and the rates of various hydrocarbon products formation were monitored using two on-line GC's. The data taken after 70 hours on-stream-time are shown in Table 1.

4

## TABLE 1

### EFFECTS OF ADDED $H_2O$ ON ACTIVITY AND SELECTIVITIES OF CO HYDROGENATION OVER 12% $Co/TiO_2$

Total Pressure = 2.1 MPa (20.7 atm.)
Feed Gas Composition = 63.9% $H_2$/32.1% CO/4.0% $N_2$
Temperature = 200°C

|  | Run 1 | Run 2 | Run 3 | Run 4 |
|---|---|---|---|---|
| SV, $SCM^3/g.hr$[*1] | 3540 | 3540 | 1180 | 1180 |
| % $H_2O$ added[*2] | 0 | 12 | 0 | 35 |
| % CO Conversion | 8.5 | 21.9 | 40.2 | 50.7 |
| $CH_4$ Selectivity[*3] | 9.6 | 4.1 | 6.5 | 2.7 |
| $CO_2$ Selectivity[*3] | 0.22 | 0.21 | 0.15 | 0.68 |
| **1-Olefin/Paraffin Ratios** | | | | |
| $C_2$ | 0.22 | 0.78 | 0.11 | 0.47 |
| $C_3$ | 2.22 | 3.92 | 1.61 | 3.29 |
| $C_4$ | 1.24 | 2.38 | 0.84 | 2.00 |
| **Hydrocarbon Product Distribution, wt. %** | | | | |
| $CH_4$ | 10.9 | 4.6 | 7.5 | 3.1 |
| $C_2$-$C_4$ | 9.8 | 5.0 | 8.1 | 4.7 |
| $C_5$-$C_9$ | 15.9 | 9.3 | 13.2 | 10.5 |
| $C_{10}+$ | 63.4 | 81.1 | 71.2 | 81.6 |
| $C_5+$ | 79.3 | 90.4 | 84.4 | 92.1 |

[*1] cc of $H_2$ and CO measured at 1 atmospheric pressure., 22°C per gram of catalyst per hour.

[*2] Moles of $H_2O$ added per 100 moles of CO and $H_2$.

[*3] Moles of $CH_4$ or $CO_2$ produced per 100 moles of CO converted.

Comparison of the data with and without the external $H_2O$ addition (Run 2 vs. Run 1 and Run 4 vs. Run 3) clearly establishes the advantages of $H_2O$ addition; (1) the productivity increases markedly (up to 2.5 fold increase), (2) the olefin selectivity increases as exemplified by the large increases in 1-olefin/paraffin ratios in the $C_2$-$C_4$ fractions and (3) the desired heavy hydrocarbon product yields, which may be gauged by the products of the Co conversion level and $C_{10}$ + selectivity, increase dramatically.

Thus, this example clearly demonstrates the benefits of conducting a Fischer-Tropsch hydrocarbon synthesis reaction with external $H_2O$ addition in the presence of a catalyst comprising cobalt supported on titania.

Example 2 - 12% Co-0.5% $Re/TiO_2$

This example demonstrates the benefits of external $H_2O$ addition when Fischer Tropsch hydrocarbon synthesis reactions are carried out using promoted $Co/TiO_2$ catalysts. This example specifically shows that the benefits of external $H_2O$ addition demonstrated in Example 1 for Fischer-Tropsch synthesis over a $Co/TiO_2$ catalyst are not diminished by addition of a promoter, or promoters, to the base catalyst, $Co/TiO_2$, as exemplified by the beneficial effects of external $H_2O$ addition to a rhenium promoted $Co/TiO_2$ catalyst.

109 grams of Co(NO$_3$)$_2$•6H$_2$O and 16 cc of aqueous perrhenic acid containing 0.83 g of rhenium (as metal) were dissolved in 300 cc acetone, to which 160 g of TiO$_2$ (rutile, BET = 13.6 $^2$/g, 80-140 mesh) was added. The acetone was then removed in a rotary evaporator, followed by further drying at 140°C in a vacuum. The resulting material was calcined in air at 250°C for 3 hours. 3 g of this catalyst was intimately mixed with 12 g of diluent (low-surface-area rutile, 80-140 mesh) and charged to the reactor as described in the previous example. The catalyst was then reduced in-situ in flowing H$_2$ (atmospheric pressure, 200 cc/m) by gradually raising the temperature from room temperature to 450°C over a period of 4 hours and then keeping at 450°C for 16 hours. The performance data, listed in Table 2, were taken after 200 hours on-stream-time.

## TABLE 2

### EFFECTS OF ADDED H$_2$O ON ACTIVITY AND SELECTIVITIES OF CO HYDROGENATION OVER RHENIUM PROMOTED Co/TiO$_2$ (12% Co - 0.5% Re/TiO$_2$)

Total Pressure = 2.1 MPa (20.7 atm.)
Feed Gas Composition = 64% H$_2$/32% CO/4% N$_2$
Temperature = 200°C

|  | Run 5 | Run 6 | Run 7 | Run 8 | Run 9 |
|---|---|---|---|---|---|
| SV, SCM$^3$/g.hr | 3510 | 3510 | 3510 | 2340 | 2340 |
| % H$_2$O added | 0 | 12 | 26 | 0 | 18 |
| % CO Conversion | 9.9 | 24.2 | 26.4 | 21.0 | 36.3 |
| CH$_4$ Selectivity | 9.4 | 4.3 | 3.4 | 8.1 | 3.6 |
| CO$_2$ Selectivity | 0.39 | 0.38 |  | 0.27 | 0.38 |
| 1-Olefin/Paraffin Ratios |  |  |  |  |  |
| C$_2$ | 0.34 | 0.48 | 0.63 | 0.22 | 0.56 |
| C$_3$ | 2.42 | 3.16 | 3.34 | 2.22 | 3.26 |
| C$_4$ | 1.71 | 1.97 | 2.39 | 1.24 | 1.97 |
| Hydrocarbon Product Distribution, wt% |  |  |  |  |  |
| CH$_4$ | 10.7 | 4.9 | 3.9 | 8.8 | 4.3 |
| C$_2$-C$_4$ | 8.4 | 4.2 | 3.6 | 5.9 | 4.0 |
| C$_5$-C$_9$ | 15.0 | 8.9 | 7.5 | 10.3 | 7.9 |
| C$_{10+}$ | 65.9 | 82.0 | 85.0 | 75.0 | 83.8 |
| C$_{5+}$ | 80.9 | 90.9 | 92.5 | 85.3 | 91.7 |

The results in Table 2 clearly establish that the benefits of external H$_2$O additions on the activity/selectivities of a rhenium-promoted Co/TiO$_2$ catalyst are comparable to those described in Example 1. This example thus establishes that promoted Co/TiO$_2$ catalysts show higher productivity and better product selectivities when external H$_2$O addition is performed during the Fischer-Tropsch hydrocarbon synthesis reaction.

Example 3

A series of runs to examine the influences of process variables, including the levels of externally added H$_2$O, were conducted using the catalyst and procedure described in Example 2, except that the catalyst used in this example was 25% less active than that described in Example 2 due to different histories. The results in Table 3 nevertheless demonstrate that the beneficial effects of external H$_2$O addition can be

realized both at a relatively low addition level of 6.7% and at a very high level of 104%. Thus, this example demonstrates that for a wide range of external $H_2O$ addition levels, 2-100% based on the combined $H_2$ and CO feeds, beneficial effects of increased productivity and desirable selectivities can be realized. The results in Table 3 also demonstrate that the beneficial effects of added $H_2O$ do not vary significantly over the temperature ranges of 180-220°C, which suggests that the effects of external $H_2O$ addition do not vary significantly with process conditions.

**TABLE 3**

**EFFECTS OF ADDED $H_2O$ ON
ACTIVITY AND SELECTIVITIES OF CO
HYDROGENATION OVER RHENIUM PROMOTED $Co/TiO_2$**

**Sum of Partial Pressures of CO and $H_2$ = 2.03 MPa (20.0 atm)
Feed Gas Composition = 64.2% $H_2$/31.8% CO/4.0% $N_2$**

| Run # | SV $SCM^3/g.hr$ | Temperature °C | % $H_2O$ Added | % CO Conversion | $CH_4$ Selectivity |
|---|---|---|---|---|---|
| 10 | 3650 | 200 | 0 | 5.2 | 13.0 |
| 11 | 3650 | 200 | 6.7 | 13.7 | 5.8 |
| 12 | 3650 | 200 | 28.0 | 18.8 | 4.1 |
| 13 | 3650 | 200 | 34.0 | 15.8 | 3.3 |
| 14 | 3650 | 200 | 104.0 | 7.7 | 2.9 |
| 15 | 10950 | 221 | 0 | 5.7 | 16.2 |
| 16 | 10950 | 221 | 25.5 | 13.9 | 7.4 |
| 17 | 1210 | 181 | 0 | 8.2 | 9.0 |
| 18 | 1210 | 181 | 23.5 | 25.4 | 2.2 |

**Claims**

1. A process for synthesising $C_5$ + hydrocarbons comprising feeding $H_2$ and CO together with $H_2O$ into contact with a catalyst composition in a reaction zone at a temperature of at least 100°C and a pressure above atmospheric, wherein said catalyst composition comprises cobalt, optionally promoted with one or more promoter metals selected from Re, Hf, Nb, Ta, Cr, Zn, and lanthanide elements, supported on titania.

2. The process of claim 1 wherein said $H_2O$ is present in an amount from 1 to 70 vol.% of the total feed of $H_2O$, $H_2$ and CO.

3. The process of claim 1 or claim 2 wherein said H2O is present in an amount from 5 to 30 vol.% of the total feed of $H_2O$, $H_2$ and CO.

4. The process of any preceding claim wherein said promoter metal is present in said catalyst composition in an amount greater than 0.01:1.

5. The process of any preceding claim wherein said promoter metal is present in said catalyst composition in an amount from 0.025:1 to 0.10:1.

6. The process of any preceding claim wherein said promoter metal is or comprises rhenium.

7. The process of any preceding claim wherein said temperature is at least 150°C.

8. The process of any preceding claim wherein said pressure is from 506 kPa to 3.04 MPa (5 to 30 atm.)

9. The process of any preceding claim wherein the mole ratio of $H_2$ to CO in said feed is from 0.5 to 6.

7

EP 0 339 923 B1

10. The process of any preceding claim wherein said catalyst composition contains from 2 to 25 % of total weight of cobalt.

**Patentansprüche**

1. Verfahren zum Synthetisieren von $C_{5+}$-Kohlenwasserstoffen, bei dem $H_2$ und CO zusammen mit $H_2O$ in eine Reaktionszone eingespeist und bei einer Temperatur von mindestens 100°C und mehr als atmosphärischem Druck in Kontakt mit einer Katalysatorzusammensetzung gebracht werden, wobei die Katalysatorzusammensetzung Kobalt, das gegebenenfalls mit einem oder mehreren Promotermetallen ausgewählt aus Re, Hf, Nb, Ta, Cr, Zn und Lanthanidelementen beschleunigt wird, aufgebracht auf Titandioxid umfaßt.

2. Verfahren nach Anspruch 1, bei dem das $H_2O$ in einer Menge von 1 bis 70 Vol.% des gesamten Einsatzmaterials aus $H_2O$, $H_2$ und CO vorhanden ist.

3. Verfahren nach Anspruch 1 oder 2, bei dem das $H_2O$ in einer Menge von 5 bis 30 Vol.% des gesamten Einsatzmaterials aus $H_2O$, $H_2$ und CO vorhanden ist.

4. Verfahren nach einem der vorhergehenden Ansprüche, bei dem das Promotermetall in der Katalysatorzusammensetzung in einer Menge von mehr als 0,01:1 vorhanden ist.

5. Verfahren nach einem der vorhergehenden Ansprüche, bei dem das Promotermetall in der Katalysatorzusammensetzung in einer Menge von 0,025:1 bis 0,10:1 vorhanden ist.

6. Verfahren nach einem der vorhergehenden Ansprüche, bei dem das Promotermetall Rhenium ist oder umfaßt.

7. Verfahren nach einem der vorhergehenden Ansprüche, bei dem die Temperatur mindestens 150°C beträgt.

8. Verfahren nach einem der vorhergehenden Ansprüche, bei dem der Druck 506 kPa bis 3,04 MPa (5 bis 30 atm) beträgt.

9. Verfahren nach einem der vorhergehenden Ansprüche, bei dem das Molverhältnis von $H_2$ zu CO in dem Einsatzmaterial 0,5 bis 6 beträgt.

10. Verfahren nach einem der vorhergehenden Ansprüche, bei dem die Katalysatorzusammensetzung 2 bis 25 % des Gesamtgewichts an Kobalt enthält.

**Revendications**

1. Procédé de synthèse d'hydrocarbures en $C_5 +$, consistant à acheminer du $H_2$ et du CO ensemble avec du $H_2O$ en contact avec une composition catalytique dans une zone réactionnelle à une température d'au moins 100°C et à une pression supérieure à la pression atmosphérique, procédé dans lequel ladite composition catalytique comprend du cobalt, éventuellement assisté par un ou plusieurs métaux promoteurs choisis parmi Re, Hf, Nb, Ta, Cr, Zn et les lanthanides, le tout étant supporté par du bioxyde de titane.

2. Procédé selon la revendication 1, dans lequel ledit $H_2O$ est présent en quantité de 1 à 70 % en volume de la charge d'alimentation totale de $H_2O$, de $H_2$ et de CO.

3. Procédé selon la revendication 1 ou 2, dans lequel ledit $H_2O$ est présent en quantité de 5 à 30 % en volume de la charge d'alimentation totale de $H_2O$, de $H_2$ et de CO.

4. Procédé selon l'une quelconque des revendications précédentes, dans lequel ledit métal promoteur est présent dans ladite composition catalytique en quantité supérieure à 0,01:1.

8

**5.** Procédé selon l'une quelconque des revendications précédentes, dans lequel ledit métal promoteur est présent dans ladite composition catalytique en quantité de 0,025:1 à 0,10:1.

**6.** Procédé selon l'une quelconque des revendications précédentes, dans lequel ledit métal promoteur est ou comprend du rhénium.

**7.** Procédé selon l'une quelconque des revendications précédentes, dans lequel ladite température est d'au moins 150°C.

**8.** Procédé selon l'une quelconque des revendications précédentes, dans lequel ladite pression est de 506 kPa à 3,04 MPa (5 à 30 atmosphères).

**9.** Procédé selon l'une quelconque des revendications précédentes, dans lequel le rapport molaire de $H_2$ à CO dans ladite charge d'alimentation est de 0,5 à 6.

**10.** Procédé selon l'une quelconque des revendications précédentes dans lequel ladite composition catalytique contient 2 à 25 % du poids total de cobalt.